# EUROPEAN PATENT APPLICATION

(11) **EP 1 310 472 A1**
(43) Date of publication of application: **14.05.2003**
(21) Application number: 02024635.1
(22) Date of filing: 04.11.2002
(51) Int. Cl.: C07C 5/27

(54) **Process of paraffin hydrocarbon isomerisation on ionic liquids as catalysts**

(30) Priority: 13.11.2001 RU 2001130402
(71) Applicant: Haldor Topsoe A/S, 2800 Kgs. Lyngby (DK)
(72) Inventor: Vladimirovna, Vasina Tamara, 109378 Moscow (RU); Modestovich, Kustov Leonid, 107392 Moscow (RU); Vladislav, Ksenofontov, Anatol'evich (RU); Egorovich, Zubarev Yurii, 143900 Moscow Region (RU); Houzvicka, Jindrich, 2840 Holte (DK); Zavilla, John, 2980 Kokkedal (DK)

(57) **Abstract**

A process for the conversion of paraffin hydrocarbon feed stock via skeletal isomerisation by contacting this feed with a catalyst comprised of an ionic liquid formed from an N-containing heterocyclic and/or N-containing aliphatic organic cation and an inorganic anion derived from metal halides.

## Description

### BACKGROUND OF THE INVENTION AND THE PRIOR ART

The present invention is related to the field of organic catalysis, in particular, to the field of catalytic isomerisation of paraffin hydrocarbons (e.g. n-heptane, n-octane etc.). The goal of isomerisation is the synthesis of high-octane motor fuels containing branched paraffin hydrocarbons.

Heterogeneous catalysts for n-paraffin isomerisation are well known and include mixed oxides, H-forms of zeolites, heteropoly acids, sulphated oxides and other systems. They are characterized by strong Broensted acidic properties. Many of these catalysts provide high yields and selectivities in the conversion of n-butane, n-pentane and n-hexane.

In the case of the isomerisation of short linear paraffins containing 4 to 6 carbon atoms, the catalysts of the Friedel-Crafts type, such as aluminum chloride, are used at relatively low temperatures (∼130°C), as well as the catalysts that contain at least one metal from group VIII supported on a halogenated, preferably chlorinated, carrier, which are used at moderate temperatures (∼150-180°C). However, the selectivity toward isomerisation products turns out to be poor in the case of the isomerisation of paraffins having more than six carbon atoms.

A number of patents describe zeolite catalysts or molecular sieves that contain at least one metal from group VIII supported onto a zeolite carrier. Such catalysts are used at high temperatures (220°C or higher) in the presence of hydrogen. In the case of linear paraffins with 5 and 6 carbon atoms, the zeolite catalysts provide negligible improvements in the octane number in the products as compared to the chlorinated catalysts, but they have the advantage of being easier to use and more resistant to poisons. As an example of the hydroisomerisation of paraffins on zeolites, the catalysts based on H-mordenite and a noble metal are described in US Patent Nos. 3,432,568 and 3,673,267. The use of H-Beta zeolites in paraffin isomerization is disclosed in US Patent No. 5,233,121 (1993), European Patent No. 398,416, US Patent No. 5,095,169 (1992), WO Application No. 96/18705 (1996) and WO Application No. 91/00851.

The isomerisation of linear paraffins having seven or more carbon atoms can also be carried out with bifunctional catalysts that combine an acid function with a hydrogenating-dehydrogenating function. The drawback of these catalysts is the occurrence of side reactions, such as cracking, hydrogenolysis, aromatisation and coke formation. The catalysts described above for the isomerisation of short linear paraffins (having 4 to 6 carbon atoms) have also been used in the hydroisomerisation of C₇ and C₈ n-paraffins, but these catalysts provide rather low yields of isomer products, because of the above side-reactions.

Another disadvantage of the known heterogeneous catalysts is the necessity to carry out the process at rather high temperature (typically above 150°C), which is not favourable for the formation of the multi-branched isomers from the thermodynamic considerations. The multi-branched isomers are characterized by high octane numbers and are valuable blending components of gasoline. Their formation in the process of isomerisation of linear paraffins becomes favourable at as low temperatures as 100°C and below.

Yet another drawback of most heterogeneous systems is that it is necessary to use hydrogen, although it is not required by the stoichiometry of the reaction equation of paraffin isomerisation. Hydrogen is used predominantly in order to prevent some side reactions and to improve the stability of the catalytic activity, for instance by minimising the coke formation. Quite a high loading of the noble metal (Pt or Pd) equal to 0.1-1.0 wt% is one more significant contribution to the cost of the catalyst and isomerisation process.

Known to the art is the catalyst for n-paraffin isomerisation (G.A. Olah, US Patent No. 4,613,723, 1986) representing a composite containing a perfluorinated alkane sulphonic acid with the number of carbon atoms in the alkyl fragment from 4 to 18, fluorinated aluminium oxide (40 wt% F) and a Lewis acid (SbF₅, TaF₅ or NbF₅). The reaction of skeletal isomerisation is carried out at a temperature of 70-80°C in the presence of hydrogen. n-Heptane or n-hexane is used as a feed hydrocarbon. Using the above catalyst, the product containing 13.1-23.4 wt% of isoheptane isomers at a selectivity of 45.5-54.2 wt% is obtained by isomerisation of n-heptane, whereas the n-heptane conversion does not exceed 43.2%. In the case of n-hexane, the yield of isohexanes is 29.7 wt% at the selectivity 50.9 wt% and conversion 58.4 wt%.

The disadvantage of this catalyst is the low activity (the conversion of n-heptane is only 43.2%) and insufficient selectivity to isoparaffins (up to 54.2% in the case of isoheptanes). Furthermore, this catalyst is extremely corrosive requiring expensive materials to avoid corrosion of the reactors and other equipment. This catalyst contains toxic compounds and elements such as fluorine (up to 40 wt%) and antimony (SbF₅), which also creates some difficulties with waste treatment. Finally, the isomerisation process is carried out under hydrogen pressure at elevated temperatures (70-80°C) and thus the process conditions are harmful, especially taking into account the corrosive properties of the system.

Yet another catalyst for isomerisation of C₄-C₁₀ paraffins, in particular, n-pentane and n-hexane, comprises a mixture of aluminum chloride and inorganic sulphate-containing matter at the atomic ratio S: Al equal to 0.1-1:1 (A. Wu, US Patent No. 5,245,103, 1993, assigned to Phillips Petroleum Company). In order to prepare the active heterogeneous catalyst composition this mixture is heated in carbon tetrachloride or dichloroethane (or other polychlorinated hydrocarbons) at 40-90°C. The reaction of n-pentane isomerisation is carried out in the presence of the thus prepared catalyst at a temperature of 33-38°C under stirring using an ultra-sound vibrator within 1-2 h. The reaction product contains 31.5 wt% of isopentane or 12.6 wt% of isohexanes at a selectivity of 72.7 wt% and 77.3 wt%, respectively.

The drawback of this catalyst is its low activity. Also, such toxic compounds, as carbon tetrachloride, chloroform, dichloroethane and other polychlorinated hydrocarbons are used in the preparation of the catalyst.

Another catalyst for paraffin hydrocarbon isomerisation, in particular, n-heptane, is based on the composition containing SbF₅ and CF₃SO₃H (trifluoromethanesulphonic acid or triflic acid) supported onto a carrier, namely, fluorinated aluminium oxide (L.E. Gardner, US Patent No. 3,878,261, 1975, assigned to Phillips Petroleum Company). For the preparation of this catalyst, the sample (13.37 g) of fluorinated aluminium oxide (39.8 wt% F) is placed into the flow reactor made of nickel and is treated with a He flow containing triflic acid. The amount of CF₃SO₃H retained by fluorinated alumina is equal to 0.0178 mole. Then the catalyst in the reactor is treated with a flow of He saturated with SbF₅ until the SbF₅ content in the catalyst reached 0.0193 mole. The resultant catalyst contains in total 33.9 wt% SbF₅ and CF₃SO₃H. The reaction of n-heptane isomerisation on this catalyst is performed at 0-24°C at atmospheric pressure in the presence of hydrogen. The n-heptane conversion achieved 47.4 wt% at the selectivity to isoheptanes of 98,2 wt%.

The disadvantages of this catalyst are as follows:
(1) The activity in n-heptane isomerisation is not very high, n-heptane conversion of 12.4-47.4 wt% is achieved.
(2) The catalytic system (CF₃SO₃H-SbF₅/Al₂O₃-F) as a result of its aggressive, corrosive, and toxic properties requires special materials for construction of the reactor and equipment, which complicates the technology and process design. Further, the utilization of wastes is another serious problem.

Recently, a new class of acidic catalysts was described in the literature: ionic liquids representing the molten salts which constitute of (1) an inorganic anion, typically formed from metal halides, such as AlCl₄⁻, Al₂Cl₇⁻, or other inorganic anions (SO₄²⁻, NO₃⁻, PF₆⁻, CF₃SO₂⁻, BF₄⁻ etc.) and (2) an organic cation, typically derived from N-heterocyclic entities (P. Wasserscheid, W. Keim, Angew. Chem., Int. Ed., 2000, V. 39, pages 3772-3789; T. Welton, Chem. Rev., 1999, V. 99, pages 2071-2083).

The melting point of ionic liquids is below 100°C and now quite a number of ionic liquids are described with melting points below room temperature. The most important advantages of ionic liquids are the following:
1. They have a liquid range of about 300°C.
2. They are good solvents for a wide range of inorganic, organic and polymeric materials.
3. They exhibit Broensted and Lewis acidity, as well as superacidity.
4. They have low or no vapour pressure.
5. Most ionic liquids are thermally stable up to near 200°C, some ionic liquids are stable at much higher temperature (about 400-450°C).
6. They are relatively cheap and easy to prepare and up-scale.
7. They are non-flammable and easy in operation.
8. They are highly polar, but non-coordinating materials.

Room-temperature ionic liquids offer promise as media for a wide range of catalytic reactions including downstream oil processing, basic organic synthesis and fine chemicals production. Among these processes of potential commercial interest are various alkylation, oligomerisation and isomerisation reactions.

Friedel-Crafts processes with ionic liquids (alkylation, acylation) are described in the literature and patents (see for example B. Ellis, F. Hubert, P. Wassersheid, WO Application No. 00/41809, 2000; A.K. Abdul-Sada, M.P. Atkins et al. US Patent No. 5,994,602, 1999; A.K. Abdul-Sada, M.P. Atkins et al. WO Application No. 95/21806, 1995; F.G. Sherif, L.J. Shyu et al. WO Application No. 98/03454, 1998). Most literature references are related to alkylation of aromatics with olefins. Nevertheless, this area is not limited by electrophilic substitution and quite a few publication describe the use of ionic liquids in alkylation of isobutane with n-butenes to produce isooctane and gasoline-range hydrocarbons (Y. Chauvin, A. Hirschauer, H. Olivier, J. Mol. Catal., 1994, v. 92, page 155). 1-Butyl-3-methylimidazoliumchloride-AlCl₃ was used as a catalyst for C₄ alkylation. 2,2,4-Trimethylpentane and dimethylhexanes were produced by alkylation and the catalyst performance was governed by the reaction conditions and composition of the ionic liquid.

Oligomerisation and polymerisation of olefins using ionic liquids as catalytic media may become a turnpike in the development of novel catalysts for olefin, especially α-olefin dimerisation, polymerisation and copolymerisation (P. Wasserscheid, W. Keim, WO Application No. 98/47616, 1998; A.K. Abdul-Sada, P.W. Ambler, WO Application No. 95/21871, 1995; Y. Chauvin, S. Einloft, H. Olivier, US Patent No. 5,550,304, 1996; Y. Chauvin, S. Einloft, H.

Olivier, US Patent No. 5,502,018, 1996; Y. Chauvin et al., French Patent No. 2,611,700).

In these studies, Ni-complexes act as catalysts and ionic liquids are considered as appropriate media capable of ideally dissolving the Ni-complexes or as co-catalysts in polymerisation. Noteworthy that the ionic liquids can be repeatedly used in oligomerisation reactions (up to 25-30 cycles without deactivation).

Thus, the available literature and patents on ionic liquids and their application in catalysis and organic synthesis show that there is an area of hydrocarbon processing, in particular n-paraffin isomerisation where the information is missing or very scarce. On the other hand, ionic liquids exhibiting the properties of strong Broensted and Lewis acids and superacidity may be promising for the application in the transformation of saturated hydrocarbons.

The object of the present invention is the development of catalysts on basis of ionic liquids possessing high activity and selectivity in isomerisation of paraffin hydrocarbons, linear and/or branched compounds forming paraffin hydrocarbons with higher degree of branching (C₄-C₁₂ isoparaffins). This catalyst should also be characterised by the low cost and it should be non-aggressive.

### DETAILED DESCRIPTION OF THE INVENTION

The object of the present invention is achieved by using the catalysts on the basis of ionic liquids for the conversion of C5-C8 paraffin hydrocarbons, linear and/or branched compounds under suitable reaction conditions leading to desirable isoparaffins.

The ionic liquids represent salts formed by an organic cation, such as N-containing heterocyclic or N-containing aliphatic moiety and an inorganic anion, which may be an anion derived from metal halides or mixed metal halides. The cation may be an alkylsubstituted pyridinium, piperidinium, quinolinium (or similar amine compounds) with one or several alkyl or aryl groups or an alkylammonium (mono, di, tri or tetra-alkyl ammonium compound). The anion may be derived from any metal halide with strong Lewis acidic properties, for instance AlCl₄⁻, AlBr₄⁻, GaCl₄⁻, Al₂Cl₇⁻, Al₂Cl₆Br⁻ and the like. The amine: Lewis acid molar ratio may be ranged from 1:3 to 2:1. Acidic properties of ionic liquids are governed by two major factors: (1) the nature of the anion, and (2) the molar ratio of the organic part to the inorganic part, for instance in the case of ionic liquids based on metal halides, Me (Hal)ₙ by the molar fraction of Me (Hal)ₙ). If X_{Me(Hal)n} < 0,5, the ionic liquid is called basic (although it is still rather acidic); if X_{Me(Hal)n} = 0,5, this is the case of neutral ionic liquid, and finally, if X_{Me(Hal)n} > 0,5, the ionic liquid can be classified as acidic or in some cases superacidic.

Ionic liquids most frequently demonstrate Lewis acidic properties, once they are formed by metal halides. In many cases, however, the ionic liquids show strong Broensted (proton) acidity. The proton acidity may originate both from the cation, if it contains a proton at the quarter-nised N atom or from the anion, if it contains protons, for instance in HSO₄⁻, H₂PO₄⁻. Also, in the case of N-heterocyclic moieties some of the protons attached to the ring may exhibit rather strong acidic properties like in the imidasolium cation.

Finally, HCl, produced via partial hydrolysis for example of the chloroaluminate anion, can explain strong proton acidity of the ionic liquids.

The effect of superacidity of ionic liquids is quite frequently observed for AlCl₃-based compositions. Sometimes, this effect is related to the presence of dry HCl in the system, which is dissolved in the ionic liquid. The Hammett function H₀ for such systems (H₀ = -18) indicates superacidic properties of the ionic liquids comparable with those of HF-TaF₅ (H₀ = -16) and "magic acid" HF-SbF₅ or FSO₃H-SbF₅ (H₀ = -25). All these systems are much stronger acids as compared to the conventional 100% H₂SO₄ (H₀ = -12), which marks the border of superacidity. Such ionic liquids are also stronger than the solid superacids like SO₄/ZrO₂ (H₀ = -16), H₃PW₁₂O₄₀ (H₀ = -13.5) or H-Nafion (H₀ = -12).

The solubility of hydrocarbons in ionic liquids is limited, and for instance paraffins and naphthenes are immiscible with ionic liquids. Olefins and aromatic compounds demonstrate a clear dependence of the solubility on the oleophilic properties of the ionic liquid. The longer the chain length of the radical attached to the N-heterocyclic moiety the higher the solubility of olefins and aromatics in the ionic liquids. However, most of the commonly used organic solvents and reagents are immiscible with ionic liquids. This simplifies the use of ionic liquids in a biphasic system and facilitates the procedure of separation.

The ionic liquid chosen for n-paraffin isomerisation may be characterized by the amine: Lewis acid molar ratio from 1:3 to 2:1, more preferably from 1:2.5 to 1:1.

Paraffin isomerisation can be carried out in an autoclave under high pressure or in a glass vessel at atmospheric pressure. The pressure in the autoclave can be varied from 0.1 MPa to 10 MPa, more preferably from 0.1 MPa to 3 MPa. Any gas like helium, argon, nitrogen, hydrogen or dry air can be used in the reaction. The reaction temperature can vary in a range from 0 to 100°C, preferably from 0 to

### 50°C. Temperatures out of this range can also be used, although they are less preferred.

Linear n-paraffins such as n-pentane, n-hexane, n-heptane, n-octane and monomethylalkanes, such as 3-methylhexane or a mixture thereof, can be used as substrates of the isomerisation process forming a product containing paraffin hydrocarbons with a higher degree of branching. In order to illustrate further the invention and the advantages thereof, the following specific examples are given.

### Example 1

The ionic liquid is prepared by slow addition of anhydrous AlCl₃ (26.67 g, 0.2 mole) to trimethylamine hydrochloride (9.56 g, 0.1 mole) in an inert atmosphere under stirring. The light-brown viscous liquid formed is heated up to 90°C and is kept at this temperature for 1 h under stirring. The resulting ionic liquid remains in the liquid state after cooling to room temperature. The similar procedure was used for preparation of other ionic liquids described in Examples 2-10 (see the Table).

### Examples 2-10

The process of paraffin isomerisation is carried out at 5-40°C using ionic liquids prepared according to Example 1. The paraffin: ionic liquid weight ratio is equal to 1:1 or 1:1.5 at atmospheric pressure in the inert atmosphere (He), while stirring the mixture with a magnetic stirrer for 1-6 h. In this experiment, a 3-neck flask with a reflux condenser connected with a gas burette is purged with helium; then the hydrocarbon starting material and 5.0 g of ionic liquid catalyst (ammoniumchloride : AlCl₃ molar ratio equal to 1:2) are loaded in the vessel and the reaction mixture is stirred for 5-6 h. The upper layer (the reaction products) is separated and analysed by gas chromatography.
The experiments 5 and 6 were carried out in a stirred autoclave (stirring rate: 300 rpm) under nitrogen (1 bar in experiment 5 and 30 bar in experiment 6).

### Examples 11-12 (comparative)

The process of n-heptane isomerisation is carried out at 0 (Example 11) or 24°C (Example 12) as described using the conventional catalyst comprising CF₃SO₃H+SbF₅ (33.0 wt%) supported onto fluorinated alumina (F content, 39.8 wt%). The results of testing are presented in the Table.

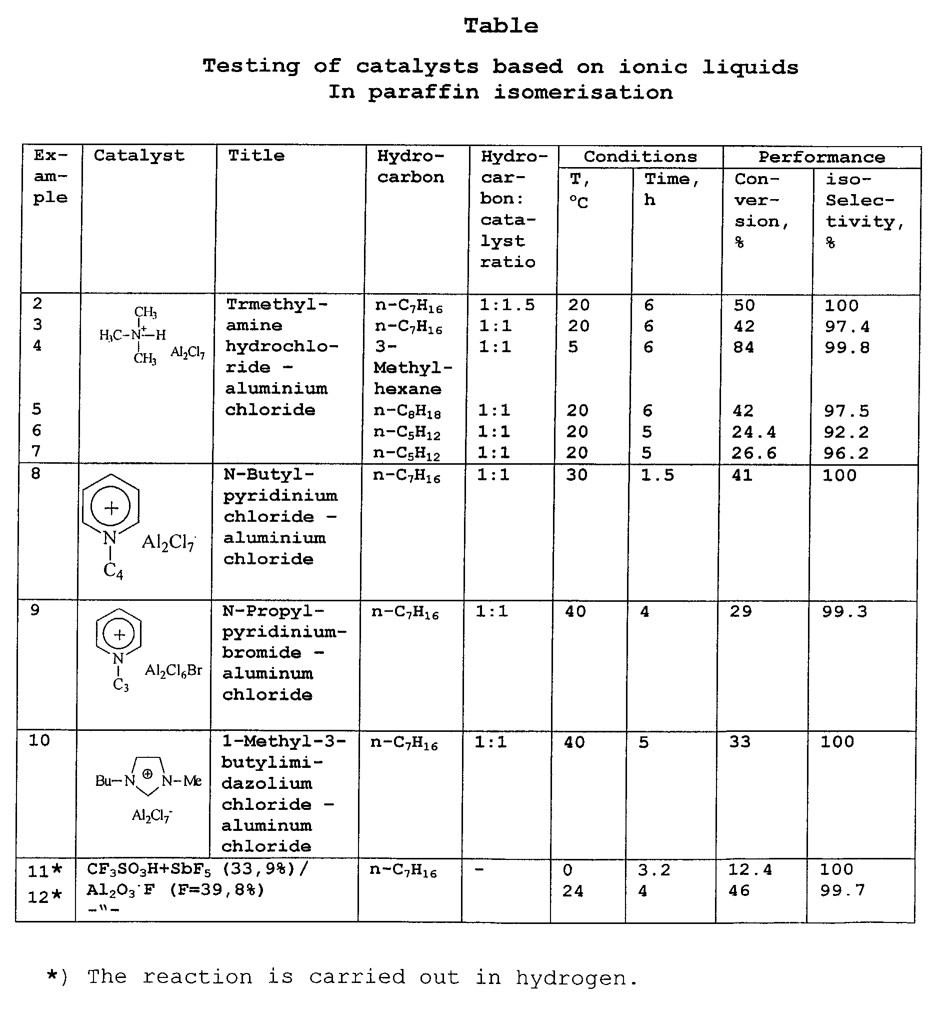

It is seen from the Table that the catalysts based on ionic liquids exhibit high activity and selectivity in isomerisation of paraffin hydrocarbons and the reaction occurs at low temperatures typically at 0-20°C. The main advantages of these catalysts are as follows:
1. They do not need to contain noble metals;
2. The reaction proceeds fast already at near-room temperature (0-20°C) unlike the conventional process on heterogeneous catalysts proceeding at higher temperatures;
3. The catalysts are cheaper and easy to prepare, further, they are less-aggressive as compared to conventional liquid acidic catalysts;
4. Since the reaction is carried out in a biphasic liquid system, the reaction products and catalyst can be easily separated and the catalyst may be repeatedly used.

## Claims

1. A process for the isomerisation of C₅-C₈ paraffin hydrocarbon feed stock in presence of an ionic liquid catalyst comprising an N-containing heterocyclic and/or N-containing aliphatic organic cation in combination with an anion derived from one or more metal halides.

2. A process of claim 1, wherein the cation of the ionic liquid catalyst is an N-aliphatic moiety with one or more alkyl or aryl groups.

3. A process of claim 2, wherein the N-aliphatic moiety is an ammonium compound and/or an alkyl substituted pyridinium, piperidinium and quinolinium.

4. A process of claim 1, wherein the metal halide is selected from AlCl₄⁻, AlBr₄⁻, GaCl₄⁻, Al₂Cl₇⁻ and Al₂Cl₆Br⁻.

5. A process of claim 1, wherein the ionic liquid catalyst is obtained by combining N-containing heterocyclic and/or N-containing aliphatic organic compounds with one or more metal halides in a molar ratio of between 1:3 and 1:0.5.

6. A process of claim 1, wherein the ionic liquid catalyst is obtained by combining N-containing heterocyclic and/or N-containing aliphatic organic compounds with one or more metal halides in a molar ratio of 1:2.
